Europäisches Patentamt
European Patent Office
Office européen des brevets

(11) Publication number: 0 282 091 A2

(12) EUROPEAN PATENT APPLICATION

(21) Application number: 88103920.0

(51) Int. Cl.4: A61L 33/00

(22) Date of filing: 11.03.88

(30) Priority: 13.03.87 US 25670

(43) Date of publication of application: 14.09.88 Bulletin 88/37

(84) Designated Contracting States: DE FR GB

(71) Applicant: BAXTER TRAVENOL LABORATORIES, INC.
One Baxter Parkway
Deerfield, IL 60015(US)

(72) Inventor: Tu,Roger H.
24755 Scott Lane
Lake Forest,California 92630(US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22(DE)

(54) **Medical device with heparin slow-release.**

(57) Materials and devices useful in blood-contacting medical applications are prepared by embedding collagen on the surface and throughout the porous interior of a synthetic polymer, covalently binding protamine to the embedded collagen, then ionically binding heparin to the protamine. The materials, and devices fabricated therefrom, are biocompatible and have prolonged anticoagulative activity due to slow release of heparin therefrom.

*FIG. 1*
BLOOD CLOTTING INDEX MEASUREMENT
FRESH HUMAN ACD-A BLOOD

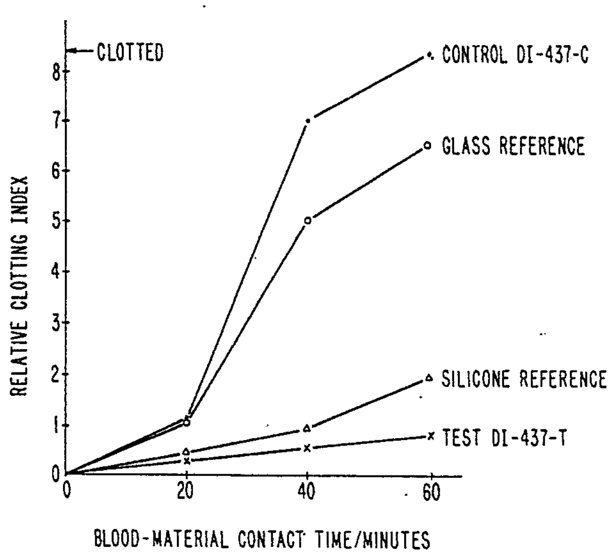


EP 0 282 091 A2

Xerox Copy Centre

# MEDICAL DEVICES WITH HEPARIN SLOW-RELEASE

## Technical Field

The present invention relates to novel materials of construction for medical devices which contact blood and methods of preparation of such materials. The materials have anti-coagulation properties which made them particularly well-suited for blood-contacting applications.

## Background of the Invention

Many types of devices which contact blood when in use are known in the medical and surgical fields. Such devices include vascular prostheses, adhesion suppressants, prosthetic ligaments, tendons, and heart valves, catheters, blood oxygenator components, dialysis membranes, arterial filters, blood reservoirs, storage containers, and many others. These devices may be used both for extracorporeal and prosthetic (surgically implanted) use. The tendency of blood to coagulate when it contacts foreign surfaces is a widely recognized problem. Much research and effort therefore have been devoted to the identification and development of materials from which medical devices can be fabricated, and which will minimize clot formation (thrombogenesis) at the material-blood interface.

Such materials have been derived from both biological tissues and synthetic fabrics, but many problems have been attendant upon their use. For example, certain materials have not proven strong or durable enough for applications such as vascular prostheses, and have been subject to tearing and ripping in use. Efforts to strengthen certain biological materials by applying a separate layer of synthetic fabric to the tissue, thus creating a laminate, have resulted in materials which present a risk of aneurysms. Many synthetic fabrics have proven to be insufficiently biocompatible for prosthetic use.

Certain materials having the anticoagulant, heparin, attached thereto by various means have been developed. However, a problem associated with many of these materials is the relatively rapid loss of anticoagulative properties, e.g., through rapid release of the heparin from the material.

Thus, a need remains for a material suitable for use in blood-contacting applications. Medical devices fabricated from such a material should not be subject to such problems as insufficient strength or durability, insufficient biocompatibility, or rapid loss of anticoagulative properties, as discussed above.

## Summary of the Invention

The present invention provides materials suitable for use in blood-contacting medical applications. The materials are strong, durable, biocompatible, and have prolonged anticoagulative properties due to the slow release of heparin therefrom. The materials comprise a porous synthetic polymer having collagen embedded both on the surface and throughout the porous interior of said synthetic polymer. Protamine is covalently bound to the collagen, and heparin is ionically bound to the protamine. The materials are prepared by embedding collagen both on the surface and throughout the porous interior of a porous synthetic polymer, then covalently binding protamine to the embedded collagen. Heparin subsequently is ionically bound to the protamine.

## Brief Description of the Drawing

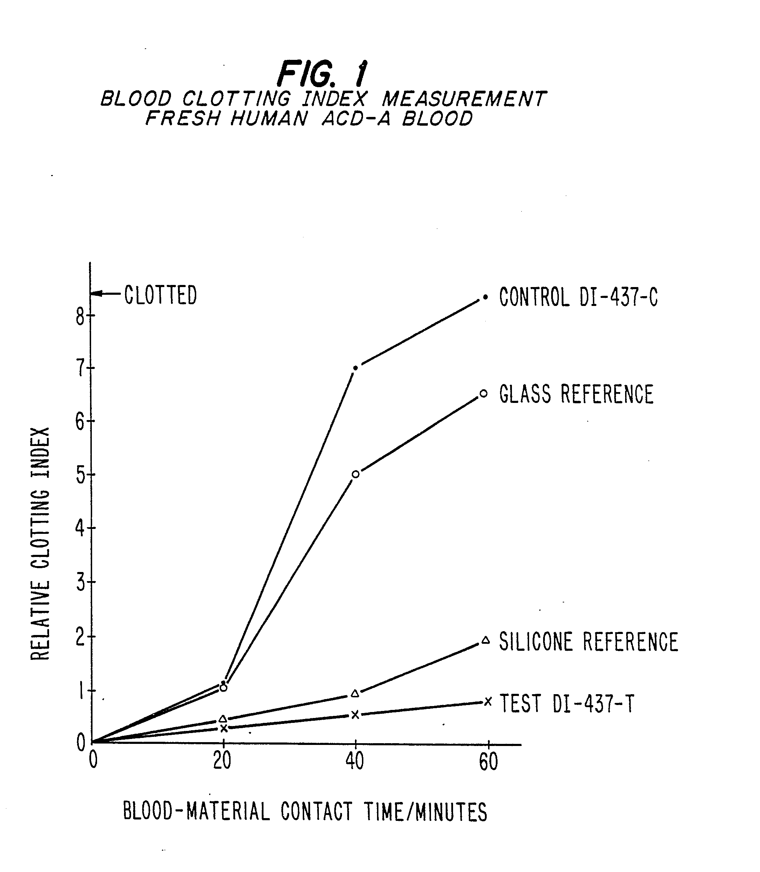

Figure 1 is a graphic representation of blood-clotting measurements of various materials over time.

## Detailed Description of the Invention

The present invention relates to a process for producing strong, durable, biocompatible materials having slow, prolonged heparin release therefrom, useful in blood-contacting applications. The process involves impregnating a porous synthetic polymer with collagen, so that the collagen is embedded both on the surface and throughout the porous interior of the synthetic polymer, covalently binding protamine to the

embedded collagen (e.g., through a crosslinking agent), and then ionically binding heparin to the protamine. The invention also relates to the novel materials produced by this process, as well as medical devices fabricated therefrom. The process of the present invention, and the advantageous properties of the devices produced thereby, are described in more detail below.

The synthetic polymer which is to be impregnated with collagen may be any biocompatable material having a degree of porosity sufficient to allow collagen to be embedded not only on the surface but also within the micropores of the polymer. The synthetic polymer should be essentially biologically inert and non-toxic, so that harmful, toxic substances will not be released from the polymer into blood. When destined for applications requiring implantation in a living host, the synthetic polymer should not cause significant adverse host reactions. The polymer also should be sufficiently strong and have the desired degree of flexibility for the particular application in which it is to be employed. For example, durability is especially important for prosthetic applications, to prevent failure of prosthetic devices or the need for frequent surgical implantation of replacement devices.

Suitable synthetic polymers (or fabrics) include polyesters, such as the polyethylene terephthalates, which are formed from terephthalic acid (or its esters) and ethylene glycol by known processes (see The Merck Index, 10th Ed., entry no. 7442. Many suitable polyesters are commercially available, including those sold under the trademarks Dacron®, Terylene®, Fortrel®, Mylar®, and Amilar®. Other suitable synthetic polymers include the polyurethanes, prepared by reaction of dihydroxy alcohols with diisocyanates, as described in Organic Chemistry, 3rd Ed. (R. Morrison and R. Boyd; Allyn and Bacon, Inc., Boston, 1977, p. 1044). Commercially available polyurethanes include those sold as Spandex® and Vycra®. Polymers derived from alkenes (e.g., polyethylene or polypropylene), polystyrene, polyvinyl alcohol, polycarbonate, inert polyolefins, silicones and polytetrafluoroethylenes also may be used. The synthetic polymer may be a homopolymer or copolymer, and polymer blends may also be employed to achieve the desired set of physical and chemical properties.

One skilled in the art will recognize the wide variety of polymers which may find use in the present invention. The particular polymer chosen may vary according to the intended application of the final product. For example, a more elastomeric polymer may be chosen when the final product is to be used as a vascular prosthesis, whereas less flexibility generally would be required for materials from which blood storage containers are to be fabricated. The degree of durability required depends on such factors as the amount of physical stress to which the final product will be subjected when in use. As discussed above, durability and strength in materials used in prosthetic devices are especially important properties.

The synthetic polymer advantageously has a porosity of about 50% or higher so that collagen can be deposited within the micropores as well as on the surface of the synthetic material. Preferred synthetic polymers for use in accordance with the present invention include fabrics woven or knitted from Dacron® polyester (prepared from methyl terephthalate and ethylene glycol and having the formula

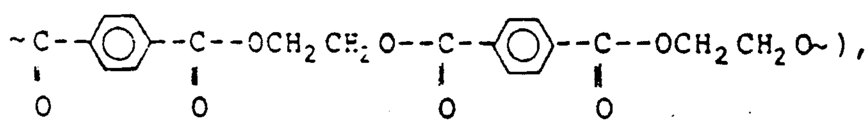

porous polyurethanes, silicones and various types of polytetrafluoroethylenes (PTFE). Many types of PTFE, and processes for their preparation, are known and are described, for example, in U.S. patents 4,332,035; 4,229,838; and 4,321,711. The microstructure of many such polymers is a network of very fine fibers and nodes connected to one another by these fibers. As described in these patents, various methods of stretching, expanding or otherwise physically treating PTFE during the preparation thereof result in a variety of polymers having porous structures which vary in porosity, pore diameter, the number and size of nodes, fiber diameter, fiber length, and other characteristics. In a preferred embodiment, the synthetic polymer is a composite of silicone and polytetrafluoroethylene.

Synthetic fabrics in certain configurations are well known, and may be employed in the present invention, depending on the intended application of the final product. For example, U.S. Patent No. 3,808,113 (column 9) mentions that polyethylene blood containers, as well as artificial blood vessels made of polyethylene, polypropylene, and polyethylene terephthalate, are all commercially available. The use of tubing made of PTFE or Dacron as vascular prostheses is discussed in U.S. Patents No. 4,229,838 (column 1), and 4,321,711 (column 1).

According to the present invention, the synthetic polymer is impregnated with collagen. The methods of collagen deposition include any means effective in depositing collagen both on the surface and into the porous interior of the synthetic polymer. Such methods include, but are not limited to, soaking, spraying,

coating, or centrifugally forcing collagen into the pores, where the collagen anchors firmly into the porous matrix of the synthetic polymer. In general, the collagen is forceably deposited within the synthetic matrix, so that collagen is embedded throughout the porous interior of the synthetic polymer. Such forced deposition of collagen into the interior matrix is particularly advantageous when hydrophobic synthetic polymers, such as PTFE, are used. For example, in one embodiment of the invention, the lumen of a tube or cylinder of synthetic polymer is filled with a solution of collagen. The solution then is pressurized to cause penetration of the fluid outwardly through the microporous wall, depositing collagen in the interior matrix of the porous polymer, and on the surface of nodes and microfibrils in fibrous synthetic polymers. Collagen also is deposited on the luminal surface.

Optionally, the embedded collagen next is fixed (tanned) using a conventional tanning agent such as formaldehyde, dialdehyde starch, or glutaraldehyde. Glutaraldehyde is a preferred tanning agent. The collagen may be fixed using any of the well-known conventional procedures. The collagen is treated with a tanning (fixing) agent under tanning conditions until proteins in the collagen are sufficiently crosslinked to strengthen and stabilize the collagen and to render it substantially non-antigenic. One suitable tanning procedure involves immersion of the collagen (embedded in the synthetic material) in a solution containing about 0.2% to 2.0%, preferably about 1.0% (w/v), glutaraldehyde in a suitable buffer (such as HEPES), having a pH of about 6.8 to 7.5. The material then is rinsed (e.g., with water, saline, or a suitable non-toxic buffer) to remove residual tanning agent.

The next step in the method of the present invention involves contacting the collagen-impregnated synthetic polymer with protamine. The synthetic polymer (having collagen embedded therein) is immersed in a solution containing protamine under conditions which allow deposition of protamine on the surface and in the porous interior of the synthetic polymer. In the case of material having a pre-formed shape such as a tube or a container, the protamine solution preferably is used to fill the tube or container, so that the protamine solution contacts the surface which will be blood-contacting during use of the final product.

Protamine is a protein of low molecular weight which is commercially available, e.g., from Sigma Chemical Company, St. Louis, Missouri, U.S.A. Protamine may also be used in the form of its salt, protamine sulfate. Protamine is a known antagonist of heparin. The concentration of protamine in the solution desirably is from about 0.1 to about 5%, most preferably about 0.5% to about 2%. The collagen-impregnated synthetic polymer is immersed in a volume of the protamine solution, and for a length of time, which together are sufficient to allow deposition of protamine on the surface and diffusion of protamine into the porous interior of the material, and adsorption of the protamine to the previously-embedded collagen. In one embodiment of the invention, the material is immersed in about 10 to about 30 mls. of protamine solution per gram of embedded collagen for about one hour. The solution should be maintained within physiologically-acceptable limits of pH and temperature to avoid damage to the collagen. Preferably, the pH of the solution is maintained between about 4.0 and about 7.0, most preferably about 5.5. The temperature of the solution preferably is maintained between about 10°C and about 30°C, most preferably about 20°C. Following immersion in the solution, the material may be washed (e.g., with water, saline, or a suitable non-toxic buffer) to remove excess protamine.

The protamine impregnated polymer then is contacted with a crosslinking agent under conditions effective in crosslinking the protamine to the previously-embedded collagen, thereby covalently binding the protamine to the collagen. Suitable crosslinking agents include formaldehyde, dialdehyde starch or glutaraldehyde. A preferred crosslinking agent is glutaraldehyde, which serves to covalently bind the protamine to the collagen. Crosslinking may be accomplished by immersing the protamine-impregnated material in a solution comprising an amount of a crosslinking agent effective to bind the protamine to the collagen. Preferably, the solution comprises about 0.2 to about 2.0% (w/v), preferably about 1.0% (w/v) glutaraldehyde. The material is immersed in the glutaraldehyde solution for a time sufficient to effect substantial crosslinking. In general, immersions for about 1/2 to about 5 hours are effective. The solution additionally may comprise a buffer. Suitable buffers include those which are biocompatible, i.e., not harmful to the collagen and also non-toxic. Suitable buffers include conventional 0.01-0.02M phosphate-buffered saline (PBS), borate, carbonate, bicarbonate, cacodylate (found to be non-toxic in animals). and other synthetic, artificial, or organic buffers such as HEPES, N-2-hydroxyethylpiperazine-N'-2-ethanesulphonic acid; MOPS, 2-(N-morpholino) propane-sulfonic acid; and PIPES, 1,4-piperazinediethanesulphonic acid.

Preferably, the buffered or unbuffered solution should not interfere with the crosslinking process afforded by the crosslinking agent such as glutaraldehyde. That is, the components of the solution should not react with the crosslinking agent or prevent the agent from achieving proper crosslinking of the protamine to the collagen. Illustrative of such unsuitable solutions are buffers containing primary and secondary amines, such as tris(hydroxymethyl)aminomethane (tris), which are known to react with the aldehyde groups of glutaraldehyde and thus may interfere with the crosslinking process. The solution also

should be maintained within physiologically-acceptable limits of pH and temperature to avoid damage (e.g., degradation or denaturation) to the collagen. After this crosslinking step, the material is washed, e.g., with water or a suitable solution, to remove excess crosslinking agent.

The final step in preparing the materials of this invention involves ionically binding heparin to the crosslinked protamine. Heparin is highly sulfated mucopolysaccharide found naturally in certain mammalian tissues, especially liver and lung. Commercial heparin preparations generally contain the sodium salt of heparin. (See The Merck Index, 10th Ed., Merck & Co., Inc., 1983, entry no. 4543, page 672).

Methods for ionically binding heparin to protamine on various vascular graft materials have been described. See, for example, Miyata et al., (Trans. Am. Soc. Artif. Intern. Organs, Vol. XXIX, pp. 363-368 [1983]) and Noishiki and Miyata (11th Annual Meeting of Soc. for Biomaterials, San Diego, Calif., April 25-28, 1985, p. 99).

The material may be contacted with heparin under conditions effective in ionically binding the heparin to the protamine. Advantageously, the material having protamine bound thereto is immersed in a solution comprising about 0.2 to about 5% (w/v) heparin, preferably about 0.5 to about 2% heparin. The heparin may be dissolved in water, saline, or alcohol. The pH of the heparin solution should be maintained between about 2 and about 8, preferably about 6.5. The material may be immersed in from about 5 to about 20 mls of the heparin solution per 5 grams of material. Preferably, the volume of solution is about 10 mls. per 5 grams of material immersed therein. The material is immersed in the solution for a length of time sufficient to allow heparin to ionically bind to the protamine both on the surface and within the porous interior of the material. Advantageously, the material is immersed in the heparin solution for about 1 to about 30 hours, preferably at least 5 hours. The temperature of the heparin solution advantageously is maintained at between about 10 and 30°C, preferably at about 20°C.

After the heparin treatment, the material is washed to remove unbound heparin, e.g., with saline, water, or the like. Medical devices having blood-contacting applications then may be fabricated from the materials. As discussed above, a wide variety of medical devices may be prepared from the materials of the present invention. Such devices include, but are not limited to, vascular prostheses (e.g., artificial arteries or other vascular grafts), adhesion suppressants, prosthetic ligaments, tendons, and heart valves, catheters, blood oxygenator components, dialysis membranes, arterial filters, blood reservoirs and storage containers, and many others, both for prosthetic and extracorporeal use. The materials of the invention may be especially useful for fabricating vascular prostheses, especially those having relatively small diameters, since prostheses for replacing or repairing such vessels are particularly prone to occlusion due to blood coagulation.

As discussed above, the materials of the present invention may be prepared using a synthetic polymer in the form of tubing as a starting material. Materials of the invention in the form of tubing are especially useful for fabricating vascular prostheses, catheters, and tubing which is part of a blood-contacting medical apparatus such as a blood-oxygenator.

The materials of the present invention, and medical devices fabricated therefrom, should be sterilized prior to use. Any suitable conventional method of sterilization may be used, as long as such method does not have deleterious effects upon any of the components of the material of the invention. Suitable methods include, but are not limited to, contacting with a sterilization-effective amount of ethanol, radiation, or glutaraldehyde.

The porosity of the synthetic starting material confers many advantageous properties upon the material prepared in accordance with the present invention. For example, the porosity of the synthetic polymer allows collagen to become embedded within the pores, i.e., the interior of the synthetic (e.g., on the nodes and microfibrils) rather than just on the outer surface. Thus, the subsequent binding of protamine, followed by heparin, occurs within the micropores of the synthetic polymer, not just on its surface. The porous synthetic polymer thus presents a large surface area for binding of heparin, since heparin ultimately is bound (through collagen and protamine, as described above) to the surfaces within the microporous matrix of the synthetic polymer. Thus, the number of sites of attachment of protamine (and subsequently heparin) are generally several times higher in the porous synthetic polymer embedded with collagen, as described herein, compared to collagen per se or to many other biologically-derived materials (optionally backed by separate layers of synthetic materials). Consequently, devices fabricated from the materials of the present invention generally have a greater amount of heparin bound thereto than would be possible with protamine, and then heparin, bound to collagen alone or to similar biologically-derived materials having known prosthetic use, such as those having a separate synthetic layer as a backing.

Another important advantage of the materials of the present invention is their slow heparin release properties. The nature of the heparin attachment (i.e., ionic bonds), the relatively large amount of bound heparin per unit (e.g., gram) of material, and the fact that heparin is located within the porous matrix of the material, combine to cause heparin release from the devices of the present invention to be quite effective

and prolonged when compared to heparin release from medical devices fabricated from other materials or having a different type of heparin binding. In accordance with a preferred embodiment of the present invention, the bound heparin is distributed evenly throughout the synthetic matrix, i.e., throughout a cross-section of the material. Thus, the diffusion or migration of the slowly-released heparin may complement the overall heparin release rate (from the protamine) to confer the prolonged heparin release properties upon the materials of the invention. The devices described herein retain anti-coagulative properties for a prolonged period, thus extending their useful life in blood contacting applications.

Another advantageous property of the materials of the present invention is the combination of strength and biocompatibility which result from the impregnation of the synthetic material with collagen, biological material. The collagen-impregnated synthetic fabric surface may be more biocompatible than the synthetic fabric surface alone. In general, the more biocompatible a material is, the less likely the material is to elicit an adverse host reaction when implanted for prosthetic use. In addition, the collagen-impregnated synthetic material has higher tensile strength, burst strength, and durability than collagen alone or than collagen backed by a separate synthetic fabric layer.

The examples presented below are provided to illustrate certain embodiments of the invention, and are not meant to limit the scope of the invention described herein.

Example 1

One piece of 6mm USCI DeBakey woven Dacron vascular graft (Cat. no. 009062) about two inches long, manufactured by C. R. Bard, Inc. was used to study how much collagen can be incorporated into its luminal surface and interstices. The water permeability was 160cc/cm$^2$. min. The collagen solution used was a 1% solution native collagen provided by Secol Company. It is an extracted fibrous animal protein coded, Secolan S-1.

The Dacron graft was soaked in said collagen solution for 30 minutes until all the air bubbles were gone. The weight of collagen solution added was 424 mg per 419 mg of porous graft. When forced impregnation outwardly took place, the weight gain remained the same. The weight gain indicated that the Dacron material is compatible with the collagen solution used.

Example 2

Collagen can be effectively deposited onto the nodes and microfibrils of an expanded PTFE throughout its matrix by forced impregnation. Due to the PTFE's hydrophobicity and microporosity, forced impregnation is a viable means to attach collagen onto its luminal surface and interstices.

A 1% Secolan S-1 fibrous native collagen was used to impregnate a 6 mm Gore-Tex PTFE vascular graft (Lot #12409-8). In a conventional impregnation, the weight gain of the collagen solution was 30.5 grams per 100 grams of PTFE matrix. When a forced impregnation was used, the weight gain of the collagen solution became 71.9 grams per 100 grams of PTFE matrix. It is clear that the forced impregnation method is helpful in incorporating more collagen onto a noncompatible porous polymer.

Example 3

Two pieces of 6 mm Gore-Tex vascular graft were force impregnated with a 1% collagen solution as in the previous example at room temperature. The collagen solution has a pH of 3.0. The samples were then fixed in a 1% glutaraldehyde solution (phosphate buffered at 7.5 pH) at room temperature for one hour, followed by rinsing in deionized water thoroughly. One sample was retained as control and coded, DI-437-C.

The other sample was impregnated in a 1% protamine sulfate solution (5.5 pH) at room temperature for one hour. It was then cross-linked in a 1% glutaraldehyde solution (phosphate buffered at 7.5 pH) at room temperature for another hour. The sample was then rinsed in deionized water thoroughly, twice.

The last step of heparin coupling was done by soaking said sample in a 1% heparin solution (6.5 pH) at room temperature for 24 hours, followed by rinsing in deionized water. This sample is coded, DI-437-T. Both DI-437-C and DI-437-T were immediately soaked in saline solution and stored in a refrigerator.

After three days of soaking, both samples, along with a glass reference and silicone reference were

subject to blood clotting time measurement. Fresh human ACD-A blood was used as a medium. Figure 1 shows the relative clotting index results which indicate that the heparinized sample, DI-437-T of this invention is the most thromboresistant surface, while the collagen coated surface, DI-437-C is the worst thromboresistant material.

**Claims**

1. A material for use in blood-contacting medical applications, comprising a porous synthetic polymer having collagen embedded both on the surface and in the porous interior of said synthetic polymer, wherein protamine is covalently bound to said collagen, and heparin is ionically bound to said protamine.

2. The material of claim 1, wherein said porous synthetic polymer has a degree of porosity sufficient to allow collagen to be embedded both on the surface and within the porous interior of said polymer.

3. The material of claim 2, wherein said synthetic polymer has a degree of porosity of about 50% or higher.

4. The material of claim 1, 2, or 3, wherein said porous synthetic polymer is selected from the group consisting of polyethylene terephthalates, polyurethanes, polyethylene, polypropylene, polystyrene, polyvinyl alcohol, polycarbonate, inert polyolefins, silicones and polytetrafluoroethylene.

5. The material of claim 1, wherein said collagen is tanned by treatment with a tanning agent under tanning conditions.

6. The material of claim 5, wherein said tanning agent is glutaraldehyde.

7. The material of claim 1, wherein said protamine is covalently bound to said collagen through a crosslinking agent.

8. The material of claim 7, wherein said crosslinking agent is glutaraldehyde.

9. A medical device having a blood-contacting application, wherein said device is fabricated from the material of claim 1.

10. The medical device of claim 9, wherein said device is selected from the group consisting of adhesion suppressants, prosthetic ligaments, tendons, and heart valves, blood oxygenator components, dialysis membranes, arterial filters, blood reservoirs, and blood storage containers.

11. The medical device of claim 9, wherein said device is in the form of tubing.

12. The medical device of claim 11, wherein said device is selected from the group consisting of vascular prostheses, catheters, and tubing which is part of a blood-contacting medical apparatus.

13. A method for preparing materials useful in blood-contacting medical applications, comprising:

a) embedding collagen both on the surface and within the porous interior of a porous synthetic polymer,

b) covalently binding protamine to the embedded collagen, and then

c) ionically binding heparin to the protamine.

14. The method of claim 13, wherein said porous synthetic polymer has a degree of porosity sufficient to allow collagen to be embedded both on the surface and within said porous interior of said polymer.

15. The method of claim 14, wherein said synthetic polymer has a degree of porosity of about 50% or higher.

16. The method of claim 13, 14, or 15, wherein said porous synthetic polymer is selected from the group consisting of polyesters, polyurethanes, polyethylene, polypropylene, polystyrene, polyvinyl alcohol, polycarbonate, inert polyolefins, silicones and polytetrafluoroethylene.

17. The method of claim 16, wherein said polymer is a composite of silicone and polytetrafluoroethylene.

18. The method of claim 13, wherein said embedded collagen is tanned by treatment with a tanning agent under tanning conditions.

19. The method of claim 18, wherein said tanning agent is glutaraldehyde.

20. The method of claim 13, wherein said collagen is embedded by a method selected from the group consisting of soaking, spraying, coating and centrifugally forcing said collagen onto the surface and into the pores of said synthetic polymer.

21. The method of claim 13, wherein said synthetic polymer is in the form of a tube, and the collagen is embedded by filling the lumen of said tube with a solution comprising said collagen, then pressurizing said solution, thereby depositing collagen on the luminal surface and in the interior matrix of said polymer.

22. The method of claim 13, wherein said protamine is covalently bound to the embedded collagen by:

(a) immersing the collagen-impregnated synthetic polymer in a solution containing protamine under conditions which allow deposition of protamine on the surface and in the porous interior of the synthetic polymer, then

(b) contacting the synthetic polymer (having collagen and protamine embedded therein) with a crosslinking agent under conditions effective in crosslinking the protamine to the embedded collagen.

23. The method of claim 22, wherein said solution containing protamine comprises about 0.1 to about 5% (w/v) protamine.

24. The method of claim 23, wherein said synthetic polymer (having collagen embedded therein) is immersed in from about 10 mls to about 30 mls of said solution containing protamine per gram of embedded collagen, for about one hour.

25. The method of claim 22, wherein said crosslinking agent is selected from the group consisting of formaldehyde, dialdehyde starch or glutaraldehyde.

26. The method of claim 25, wherein said crosslinking agent is glutaraldehyde, and said synthetic polymer having collagen and protamine embedded therein is immersed in a solution comprising from about 0.2 to about 2% (w/v) glutaraldehyde for from about 1/2 to about 5 hours.

27. The method of claim 13, wherein heparin is ionically bound to the protamine by immersing said polymer (having said protamine covalently bound to the collagen embedded therein in a solution comprising from about 0.2 to about 5% (w/v) heparin.

28. The method of claim 27, wherein said polymer is immersed in the heparin solution for about 1 to about 30 hours.

29. The method of claim 27, wherein said polymer is immersed in from about 5 to about 20 mls of the heparin solution per 5 grams of polymer.

30. The method of claim 27, wherein the pH of the heparin solution is from about 2 to about 8.

31. The method of claim 27, wherein said polymer is immersed in about 10 mls per 5 grams of polymer of a solution comprising about 1% (w/v) heparin, and having a pH of about 6.5 for about 24 hours.

*FIG. 1*

*BLOOD CLOTTING INDEX MEASUREMENT*
*FRESH HUMAN ACD-A BLOOD*